# EUROPEAN PATENT APPLICATION

(11) **EP 4 565 016 A1**
(43) Date of publication of application: **04.06.2025**
(21) Application number: 24215627.1
(22) Date of filing: 27.11.2024
(51) Int. Cl.: H05K 3/32, H05K 1/02, H05K 3/00, B29C 65/00, H05K 3/34, H05K 1/18

(54) **AUTOMATED CONTROL OF AN ELECTRICAL CONNECTION JOINING PROCESS**

(30) Priority: 28.11.2023 US 202363603144 P; 20.09.2024 US 202418890889
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); SITNITSKY, Ilya, 2066717 Yokneam (IL); SHECHTMAN, Alexander, 2066717 Yokneam (IL); FUCHS, Amit, 2066717 Yokneam (IL)
(74) Representative: Carpmaels & Ransford LLP

(57) **Abstract**

A method of electrically connecting a wire to a connection pad of a flexible circuit strip using a heat joining tool, wherein the flexible circuit strip includes a polymer body, a plurality of electrodes, a plurality of connection pads and a plurality of conductive traces, a trace connecting each of the plurality of electrodes to a connection pad of the plurality of connection pads, the method including: positioning the joining tool in proximity to a connection pad of the plurality of connection pads and a wire to be connected to the connection pad; activating the joining tool to heat a tip of the joining tool to electrically connect the wire to the connection pad; monitoring, during the activating, a resistance between the joining tool and an electrode electrically connected to the connection pad, which resistance indicative of temperature of the flexible circuit strip; and controlling the activating to maintain the resistance below an upper threshold associated with the flexible circuit strip reaching a damaging temperature.

## Description

### RELATED APPLICATIONS

This application claims priority from U.S. Provisional Application No. 63/603,144, filed on 28 November 2023, the contents of which is incorporated by reference as if fully set forth herein.

### TECHNOLOGICAL FIELD

The present disclosure, in some embodiments thereof, relates to catheter manufacture and, more particularly, but not exclusively, to establishing electrical connections to a flexible circuit strip of the catheter.

### BACKGROUND

A wide range of medical procedures involve placing probes, such as catheters, within a patient's body. Electrode catheters have been in common use in medical practice for many years. They are used to stimulate and map electrical activity in the heart and to ablate sites of aberrant electrical activity.

A wall cavity of an organ of a patient, such as a cardiac cavity, can be mapped and/or ablated using a catheter having multiple electrodes fitted at an expandable distal end assembly of the catheter. The expandable distal-end assembly is coupled at a distal end of a catheter shaft for insertion into the cavity. The expandable distal end assembly may be shaped in the form of a balloon, basket and/or another type of cage and may include a plurality of electrodes configured for sensing and/or delivering therapeutic signals.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
FIG. 1A is a schematic view of a basket catheter, according to some embodiments of the disclosure;
FIG. 1B is a simplified schematic of flexible polymer circuit strips, according to some embodiments of the disclosure;
FIG. 2 is a simplified schematic of a system for establishing electrical connections to a flexible circuit strip, according to some embodiments of the disclosure;
FIG. 3A is a method of establishing a connection to a workpiece, according to some embodiments of the disclosure;
FIG. 3B is a method of establishing a connection to a workpiece, according to some embodiments of the disclosure;
FIG. 4 is a simplified schematic plot of resistance with time, according to some embodiments of the disclosure; and
FIGs. 5A-E are simplified schematics illustrating establishing electrical connection to a pad using a joining tool, according to some embodiments of the disclosure.

In some embodiments, although non-limiting, like elements are referred to using like numerals.

### DETAILED DESCRIPTION OF EMBODIMENTS

The present disclosure, in some embodiments, thereof, relates to catheter manufacture and, more particularly, but not exclusively, to establishing electrical connections to a flexible circuit strip of the catheter.

### Overview

Some catheters, such as basket catheters, may have many (e.g., 30-100) electrodes mounted on a plurality of splines of the basket, which is coupled through a shaft of the catheter to a connector at the proximal end of the shaft. In some embodiments, the electrodes may be hosted by a plurality of flexible printed circuit boards (PCB) each of which is mounted on a spline of the basket. Both the flexible PCBs, also herein termed "flexible circuit strip" on the splines and a PCB of the connector have pads and a plurality of wires, e.g., a wire for each electrode, running through the shaft, provides electrical coupling. The distal end of each wire is coupled to a predefined pad on the spline, and a proximal end of each wire is coupled to a corresponding pad in the connector, so as to exchange electrical signals between the electrodes and a control console, e.g., a PIU (patient interface unit) of the catheter, via the shaft. Due to the limited real estate on the splines, the pads are required to cover a small area. For example, the pad size, (e.g., pad surface area) may be in the order of magnitude of tens of micrometers squared, (also referred to herein as microns squared) for each pad on the printed circuit board, resulting in a potentially time-consuming and/or costly coupling process between the wires and the respective pads. Pads may also be placed close to one another e.g., spacing between pads being less than pad length and/or width and/or where there is less than 0.1-0.5 mm between adjacent pads. The pads may densely occupy an area on the flexible circuit strip, where, for example, a proportion of the surface area occupied by pads is 20-50%.

In manufacture, heat joining (e.g., soldering or welding) of the many wires to the many pads of a flexible circuit strip (e.g., sequentially), e.g., involving repeated heating of wires and pads, may increase temperature of the flexible circuit strip e.g., to a level at which material of the strip (e.g., a polymer body of the flexible circuit strip) suffers damage. Associated, for example, with repeated heating for connection of multiple pads, heat applied to connect a wire to one pad may lead to undesired heating of another neighboring pad that has already been connected to a wire e.g., further affecting the polymer body around the previously connected and heated pad and/or potentially degrading a quality of connection which as already been established.

A broad aspect of some embodiments of the disclosure relates to monitoring resistance between a joining tool connecting a wire to a pad and an electrode corresponding to the pad. The resistance and/or changes to the resistance over time potentially providing an indication as to temperature of the flexible polymer strip, e.g., where increasing resistance generally indicates increasing temperature. For example, as resistance detected includes that of the pad, the electrode, and the tracing connecting the pad and electrode, each of which may be affected by temperature.

An aspect of some embodiments of the disclosure relates to establishing electrical connection to a pad of a flexible circuit strip using a joining tool where heating of the joining tool for forming of the connection is controlled using detected resistance between the joining tool and an electrode connected to the pad.

In some embodiments, the flexible circuit strip includes material which is heat-sensitive (e.g., includes polymer). Heat-sensitivity may be manifested as deformation and/or chemical change/s to the material by exposure to temperatures over a threshold and/or raised temperatures for longer than a threshold duration of time.

The resistance detected is potentially indicative of temperature of the polymer body of the flexible polymer strip, which may be prone to increasing in temperature during the connection process, the increase associated with thermally insulating nature of the polymer material e.g., in comparison to the electrical components.

In some embodiments, the flexible circuit strip includes a plurality of pads, and the establishing is of a plurality of connections to each of the plurality of pads, for example, sequentially and/or by a single joining tool.

In some embodiments, the pads are closely packed and/or directly disposed on the heat-sensitive material e.g., the polymer body. In some embodiments, electrical circuitry connected to the pads (e.g., trace and electrode for each pad) conduct heat from the pads to other regions of the flexible circuit strip, heat at the pad potentially increasing temperature of polymer portions elsewhere e.g., adjacent to trace/s and/or electrode/s.

In some embodiments, heating of the joining tool during the joining process is non-continuous. Where, for example, a power supply to heating elements is a pulsed power supply (e.g., including voltage pulses). Without wanting to be bound by theory, it is theorized that the cycles of heating and cooling enable heating of the elements which are to be connected (e.g., one or more of the pad, wire to be connected to the pad, and solder material) to rise to temperatures suitable for establishing connection while heat-sensitive portion/s of the flexible circuit strip remain below a temperature at which damage may occur. It is theorized that high thermal conductivity of the element/s (e.g., metal) being connected as compared to low thermal conductivity of other portion/s of the flexible circuit strip (e.g., polymer) enable cycles of heating and cooling to raise temperature of the element/s to be connected while minimally raising temperature of the heat-sensitive portions.

In some embodiments, one or more feature of the pulsed power supply is controlled using resistance levels detected. For example, where the feature/s include one or more of pulse duration (width), amplitude, and temporal pattern (e.g., frequency). In some embodiments, control of pulsed power supply to the heating element/s is by pulse width modulation (PWM). In some embodiments, resistance is detected during the joining process e.g., providing feedback for control of the power supply to the heating element/s during the joining process.

In some embodiments, the control is to maintain the resistance below an upper threshold resistance, for example, where the upper threshold resistance is associated with damaging temperatures for the flexible circuit strip.

In some embodiments, the control is to ensure quality of the joining process (e.g., quality of the heating). Where, if the quality of heating is identified as being low, power supply (e.g., amplitude of voltage pulses to the heating element/s) is increased. In some embodiments, quality of the joining process is ensured by maintaining the resistance above a lower threshold resistance, for example, where the threshold is associated with temperatures for which the joining process is efficient and/or effective. Alternatively or additionally, in some embodiments, quality of the joining process is ensured by maintaining a rate of change of resistance during heating (e.g., during a power supply pulse) above a threshold rate.

In some embodiments, the threshold/s are determined by detecting of levels of resistance at different flexible circuit strip temperatures, for example, for a plurality of flexible circuit strips and/or electrodes and pads thereof. Where, in some embodiments, average values are used to determine the threshold/s.

In some embodiments, resistance is additionally detected between the joining tool and the pad which is being connected. In some embodiments, this "pad resistance" is used to determine a portion of (e.g., sum portion of) the "electrode resistance" (resistance detected between the joining tool and electrode) which is associated with temperature of the flexible circuit strip, independent of the pad. In some embodiments, the pad resistance is monitored during the joining process where both types of resistance detected are used in control of the power supply to the heating elements. Alternatively, in some embodiments, pad resistance is detected during a data collection process e.g., as described above regarding determining thresholds. Where, in some embodiments, the thresholds are determined using both types of resistance, but where the thresholds themselves correspond to the electrode resistance only.

An aspect of some embodiments of the disclosure relates to identifying when a sufficient quality electrical and/or mechanical connection has been established between a pad and wire. Where, upon the identifying, heating of the joining tool may be stopped. A potential advantage of accurate and/or early identification of establishment of a sufficiently good connection enables a reduced duration of heating and/or reduced heating temperature.

In some embodiments, from one or both of the pad resistance and the electrode resistance, a drop in resistance is identified. Where the drop is associated with establishment of the connection e.g., and not with cooling. For example, where, in some embodiments, the drop is identified during a power supply pulse to the heating element/s of the joining tool.

Alternatively, or additionally, in some embodiments, an initial resistance detected (pad and/or electrode) before an initial activation of the joining tool is compared to a resistance detected after a joining process and after the flexible circuit strip has cooled to an initial temperature. Where the drop in resistance is identified in this comparison. In some embodiments, if this drop is insufficient, the joining process may be repeated e.g., to increase mechanical and/or electrical quality of the connection.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary catheter

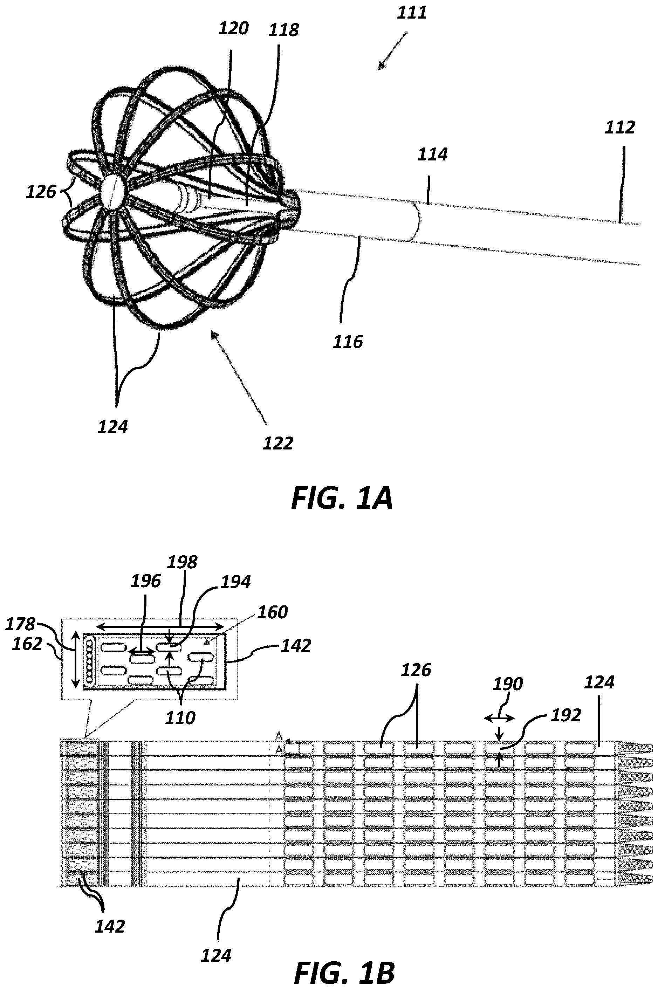

FIG. 1A is a schematic view of a basket catheter 111 according to some embodiments of the disclosure.

The basket catheter 111 includes an elongated deflectable element 112 having a distal end 114, a coupler 116 connected to the distal end 114, and an optional pusher 118. The optional pusher 118 is configured to be advanced and retracted through the deflectable element 112, for example, using a manipulator or handle (not shown). The basket catheter 111 also includes an expandable assembly 122 comprising a plurality of flexible circuit strips 124 (only some labeled for the sake of simplicity) also herein termed "flexible splines". Where, in some embodiments, the flexible circuit strips 124 are polymer circuit strips. Each flexible circuit strip 124 includes multiple electrodes 126 disposed thereon (only some labeled for the sake of simplicity). The formation of the various elements and how they are connected with each other are described in more detail in US Patent Application Publication No. US 2021/0187241.

In some embodiments, form and/or structure of basket catheter 111 is provided by a basket structure, which in some embodiments, includes (e.g., is formed of) nitinol e.g., including a plurality of nitinol strips (also herein termed "splines"). Where a least a portion of flexible circuit strips 124 are each supported by a corresponding nitinol strip. In some embodiments, strips of the nitinol basket structure are connected together e.g., at a proximal end of basket catheter 111. Flexible circuit strips 124 may be mounted to the nitinol basket structure prior to or after establishing electrical connections to the pads (e.g., pads 110 FIG. 1B) of flexible circuit strips 124. In some embodiments, electrical connections to pads 110 of flexible circuit strips 124 are established when the flexible circuit strips 124 are in a flat and/or un-disconnected configuration e.g., the configuration illustrated in FIG. 1B, prior or after connecting of the flexible circuit strips to the nitinol support structure.

### Exemplary flexible circuit strip embodiments

FIG. 1B is a top view of flexible polymer circuit strips 124 prior to their assembly for use in the basket catheter of FIG. 1A.

Reference is now made to FIG. 1B, which is a schematic view of the flexible polymer circuit strips 124 for use in the basket catheter 111 of FIG. 1A. The flexible polymer circuit strips 124 may be formed from a single piece of polymer, such as polyimide. Circuit strips 124 may be connected to each other by polyimide, or assembled as individual pieces that are held in proper alignment and secured to coupler 116 FIG. 1A. By manufacturing circuit strips 124 as individual components the yield of the base circuit may be increased as a failed electrode scraps one circuit strip rather than an entire assembly of strips. Respective first ends 142 of the respective flexible polymer circuit strips 124 include an electrical connection array 160.

An inset 162 shows that the electrical connection array 160 includes pads 110 thereon (only some labeled for the sake of simplicity). The pads 110 are connected via traces (not shown), optionally located on the back of the flexible polymer circuit strips 124, to respective ones of the electrodes 126 disposed on the front of the flexible circuit strips 124. Away from the region of the first ends 142, the flexible circuit strips 124 are separate from each other to allow the flexible circuit strips 124 to form the expandable assembly 122 (FIG. 1A) when connected to the basket catheter 111. Wires (not shown) may connect the electrodes 126 to control circuitry (not shown) via pads 110. The wires may be disposed in lumens (not illustrated) of the elongated deflectable element 112 (FIG. 1A).

The flexible circuit strips 124 may have any suitable dimensions. For example, the length of the flexible circuit strips 124 may be in the range of 10 mm to 60 mm, e.g., 30 mm, the width of the flexible circuit strips 124 may be in the range of 0.25 mm to 3 mm, e.g., 0.72 mm, the thickness of the flexible circuit strips 124 may be in the range of 0.005 mm to 0.14 mm.

In some embodiments, electrodes 126 extend to occupy a majority of the width of the flexible circuit strips 124 by which they are hosted. Where, in some embodiments, electrode width 192 is 0.25-3 mm, and/or electrode length 190 is 0.25-3 mm.

In some embodiments, one or more pad dimensions are smaller than electrode dimensions. Where the surface area the pad presents for contact (e.g., to a measurement probe or joining tool) is smaller than that presented by the electrodes. For example, a pad upper surface area being 1-20% of that of the electrodes. For example, a pad width 194 being 0.05-0.5 mm, and/or a pad length 196 0.1-1 mm.

A surface area 162 of the flexible circuit strips 124 on which pads 142 are disposed may be small and/or the density of pads on the area may be high. For example, the area having a width 178 of 0.25-3 mm and a length 198 of 0.5-6 mm and/or the proportion of the surface area occupied by pads is 20-50%.

### Exemplary electrical connection system

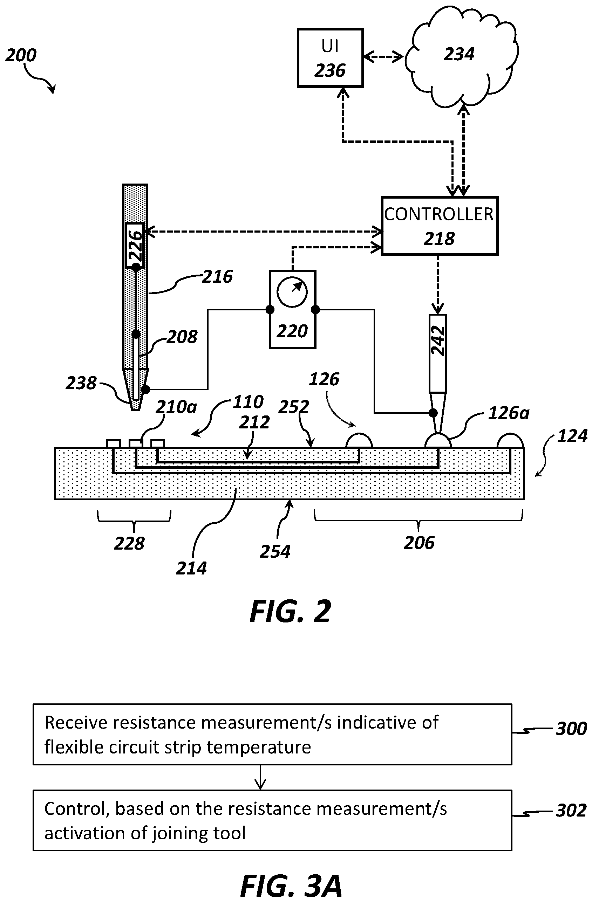

FIG. 2 is a simplified schematic of a system 200 for providing electrical connections to a flexible circuit strip 124, according to some embodiments of the disclosure.

In some embodiments, flexible circuit strip 124 is a portion of a catheter e.g., having one or more features as illustrated in and/or described regarding catheter 111 FIG. 1A. Where, in some embodiments, flexible circuit strip 124 corresponds to one or more (e.g., each) of the flexible circuit strips 124 of FIG. 1A and/or FIG. 1B.

In some embodiments, system 200 includes a joining tool 216 configured to join an electrical connector e.g., a wire (not illustrated) to each of pads 110 (e.g., one pad at a time). In some embodiments, joining tool 216 is a heat joining tool which joins by generating and supplying heat to the object/s to be joined e.g., soldering iron, or welding gun, or ultrasonic joining tool.

In some embodiments, system 200 includes one or more sensor 220 where sensor/s 220 are resistance sensors. In some embodiments, resistance sensor 220 detects resistance between joining tool 216 and an electrode (e.g., electrode 126a as illustrated in FIG. 2). In some embodiments, a probe 242 is used to provide electrical contact to the electrode. Optionally, (not illustrated) in some embodiments, resistance is detected directly between joining tool 216 and a pad 210a e.g., by contacting the pad.

In FIG. 2, in some embodiments, electrical connections (e.g., for detection of resistance) are illustrated by solid lines, and data connections (e.g., transfer of detected resistance levels to controller 218) are illustrated by dashed lined arrows.

In some embodiments, joining tool 216 is controlled by a controller 218 which receives sensor signals from one or more sensor. For example, from one or more of resistance sensor/s 220.

In some embodiments, controller 218, for example, based on detected resistance levels (e.g., from sensor/s 220) generates control signals for activation of joining tool 216. In some embodiments, controller 218 generates and sends control signal/s to a power supply 226 of joining tool 216, where the control signal/s control power supplied by power supply to heating element/s 208. Alternatively or additionally, in some embodiments, controller 218 generates control signal/s which are translated (e.g., by a joining tool) processor into control signals for power supply 226.

In some embodiments, power supply 226 is configured to supply pulses of power to heating element/s 208, where, in some embodiments, based on the detected resistance, one or more features of the pulses (e.g., one or more of amplitude, pulse repetition frequency, and pulse duration) are controlled based on the detected resistance. In some embodiments, pulse width modulation (PWM) is used.

Optionally, in some embodiments, system 200 includes one or more temperature sensor (not illustrated), for example, the joining tool may include a temperature sensor which may provide temperature feedback measurement/s to controller 218 for control of temperature of the joining tool.

In some embodiments, system 200 includes a rigid support (not illustrated) which provides mechanical support to flexible circuit strip 124 e.g., enabling joining tool 216 to apply pressure to a pad e.g., during a joining process

In some embodiments, flexible circuit strip 124 has at least one (e.g., a plurality) of electrodes 126. Where electrodes 126 may be disposed on a distal portion 206 of flexible circuit strip 124. Electrodes 126 may be disposed on an external surface of flexible circuit strip 124 e.g., one or more of electrodes 126 may protrude from a generally planar external surface (e.g., a top surface) of flexible circuit strip 124 and/or one or more of electrodes 126 may have an electrode top surface flush (or recessed from) the flexible circuit strip 124 top surface.

In some embodiments, a plurality of pads 110 are disposed on a proximal portion 228 of flexible circuit strip 124. In some embodiments, one or more of electrodes 126 e.g., each electrode, is electrically connected to a corresponding pad of plurality of pads 110 e.g., each electrode to a pad by an individual trace 212, flexible circuit strip 124 including a plurality of traces 212. In some embodiments, the electrical connections (e.g., traces or wires) extend through a body 214 of flexible circuit strip 124.

In some embodiments, flexible circuit strip 124 includes a plurality of layers where, for example, wires 212 connecting pads 110 to electrodes 126 are sandwiched between at least two layers. Where, in some embodiments, an outer surface 252 of flexible circuit strip 124 (also herein termed "top surface" 252) hosts pads 110 and electrodes 126. In some embodiments, top surface 252 includes electrically insulating material (e.g., portions which are not the pads or electrodes are formed of polymer).

Alternatively, or additionally, in some embodiments, the traces are disposed on a back surface 254 of flexible circuit strip, where, for example, connectors extend through body 214 to connect pads and electrodes to respective traces. Where back surface 254, in some embodiments, is a surface of body 214 generally opposing top surface 252.

In some embodiments, joining tool 216 is automatically moveable with respect to flexible circuit strip 124 (e.g., by one or more actuators/s) for moving the joining tool between locations of the plurality of pads. Where, in some embodiments, one or both of joining tool 216 and the support on which the flexible circuit strip is disposed are moveable with respect to each other. In some embodiments, positioning of joining tool 216 with respect to the flexible circuit strip 123 is controlled by controller 218.

In some embodiments, the joining tool tip 238 includes an outer surface e.g., a cover including material which is highly electrically and/or thermally conductive and/or heat resistant. For example, the joining tool including a cover configured to cover (e.g., surround) at least a portion of joining tool 216 between a region of electrical contact between resistance sensor/s 220 and joining tool 216. In some embodiments, (e.g., as associated with high electrical and/or thermal conductivity) resistive and/or capacitive properties of the joining tool have low dependence on temperature.

In some embodiments, at least a portion of the joining tool which contacts a pad (e.g., tip 238 and/or a portion of the tip) during establishing connection to the pad is non-adhesive to material of pads 110. For example, at temperatures employed during establishing of connections to pads. When the joining tool is a soldering iron, material of the joining tool which contacts solder may be non-adhesive to solder e.g., alternatively or additionally to being non-adhesive to the pad surface.

In some embodiments, the joining tool tip and/or cover and/or region of the joining tool in an electrical path between the joining tool and sensor/s 208, includes (e.g., is formed of) gold.

In some embodiments, user instructions (e.g., to initiate the joining process) are received through and/or information is displaced to a user through one or more user interface (UI) 236. Where, in some embodiments, data is transferred between UI 236 and controller 218. For example, in some embodiments, user instructions are transferred by UI 236 to controller 218 which, in some embodiments, controls operation of system 200 based on the user instructions. One or more UI may be local to joining tool 216 and/or other system components and/or one or more UI may be located remotely.

Optionally, in some embodiments, external processing and/or memory circuitry 234 communicates with UI 236 and/or controller 218. For example, circuitry 234 enabling remote control of system 200 e.g., via remote UI/s.

### Exemplary electrical connection method

FIG. 3A is a method of establishing a connection to a workpiece, according to some embodiments of the disclosure.

At 304, in some embodiments, resistance between the joining tool and the electrode is detected.

At 306, in some embodiments, activation and/or temperature of the joining tool is controlled based on the detected resistance level/s. In an exemplary embodiment, activation of the joining tool is controlled by controlling power supplied to heating element/s of the joining tool.

In some embodiments, heating of the joining tool during the joining process is non-continuous. Where, for example, a power supply to heating elements is a pulsed power supply (e.g., including voltage pulses).

In some embodiments, one or more feature of the pulsed power supply is controlled using the detected resistance. For example, where the feature/s include one or more of pulse duration (width), amplitude, and temporal pattern (e.g., frequency). In some embodiments, control of pulsed power supply to the heating element/s is by pulse width modulation (PWM). In some embodiments, the resistance is detected during the joining process e.g., providing feedback for control of the power supply to the heating element/s during the joining process.

In some embodiments, the control is to maintain the resistance below an upper threshold resistance, for example, where the threshold is associated with the flexible circuit strip reaching damaging temperatures.

Optionally, in some embodiments, the control is to ensure quality of the joining process (e.g., quality of the heating). Where, if the quality of heating is identified as being low, power supply (e.g., voltage e.g., amplitude of voltage pulses to the heating element/s) is increased. In some embodiments, quality of the joining process is ensured by maintaining the resistance above a lower threshold resistance, for example, where the threshold is associated with temperatures for which the joining process is efficient and/or effective. Alternatively or additionally, in some embodiments, quality of the joining process is ensured by maintaining a rate of change of resistance during heating (e.g., during a power supply pulse) above a threshold rate.

In some embodiments, the threshold/s are received e.g., from a memory (e.g., of circuitry 234). In some embodiments, the threshold/s are provided by a user e.g., thorough a user interface. In some embodiments, the thresholds are determined by detecting resistance while heating the flexible circuit strip to different temperatures. For example, detecting resistance levels at different flexible circuit strip temperatures, for example, for a plurality of flexible circuit strips and/or electrodes and pads thereof. In some embodiments, averages are used to determine the threshold/s.

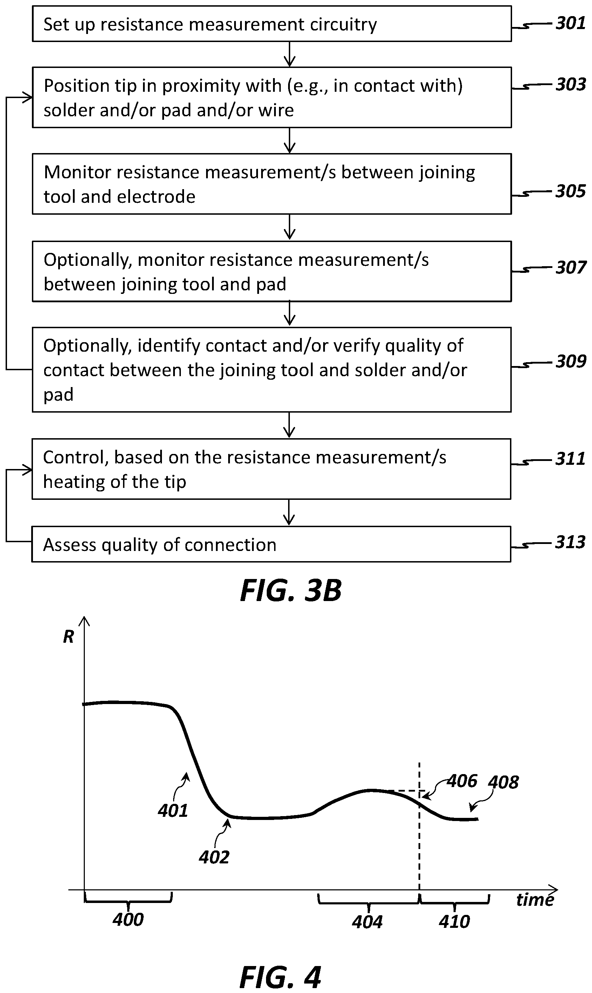

FIG. 3B is a method of establishing a connection to a workpiece, according to some embodiments of the disclosure.

At 301, in some embodiments, resistance measurement circuitry is set up. For example, where a measurement probe is brought into contact with an electrode corresponding to the pad (e.g., probe 242, electrode 126a, and pad 210a, FIG. 2). In some embodiments, resistance measurement circuitry has fixed position e.g., connection to the electrode for determining resistance being provided by a jig where, for example, positioning the flexible circuit strip for the electrical connection process sets up the resistance measurement circuitry.

At 303, in some embodiments, a tip of a joining tool (e.g., 238 tip of joining tool 216 FIG. 2) is brought into proximity (e.g., contact with) a pad and/or a wire to be connected to the pad and/or solder (e.g., where the joining tool is a soldering iron). In some embodiments, the proximity is sufficient for enough heat to be conducted from the joining tool to the pad and/or wire and/or solder for the connecting process e.g., melting of soldier and/or pad and/or wire material. In some embodiments, positioning of the joining tool is an automated process, for example where one or more actuator controlled by a controller (e.g., controller 218 FIG. 2) is configured to move the joining tool with respect to the flexible circuit strip.

At 305, in some embodiments, electrode resistance is monitored. For example, according to one or more feature of step 300 FIG. 3A.

At 307, optionally, in some embodiments, resistance is additionally monitored between the joining tool and the pad which is being connected. In some embodiments, this "pad resistance" is used to determine a portion of the "electrode resistance" (resistance detected between the joining tool and electrode at step 305) which is associated with temperature of the trace and electrode, independent of the pad e.g., indicative of the temperature of the flexible circuit strip.

In some embodiments, both resistances are monitored during the joining process and used in control of the power supply to the heating elements.

Alternatively, in some embodiments, pad resistance is detected during a data collection process e.g., as described in step 302 determining thresholds. In some embodiments, the thresholds are determined from data collection of both types of resistance detected, but detected electrode resistance level/s (e.g., only) are used during the joining process.

At 309, optionally, in some embodiments, the detected electrode and/or pad resistances are used to identify contact between the joining tool and pad and/or verify that quality of the contact is sufficient. Where, in some embodiments, contact is identified as a drop in resistance (e.g., slope of the drop in resistance being above a threshold) detected and/or as the resistance detected falling to below a threshold.

If contact is not verified and/or quality of the contact is not verified (e.g., resistance detected is higher than a threshold) the tip may be repositioned e.g., returning to step 303. Where, for example, the tip may be moved towards the pad e.g., to increase pressure between the pad and the tip to improve electrical and/or thermal contact therebetween.

At 311, in some embodiments, heating of the joining tool tip is controlled, using the detected resistance. Where the electrode resistance may be used, e.g., according to one or more feature of step 302 FIG. 3A and, optionally, where pad resistance may be additionally used.

At 313, in some embodiments, a quality of connection between the wire and pad is assessed using detected resistance. For example, in some embodiments, a drop in resistance is identified in one or both of the pad resistance and the electrode resistance. The drop may be associated with establishment of electrical connection e.g., and not with cooling. For example, where, in some embodiments, the drop is identified during a power supply pulse to the heating element/s of the joining tool. In some embodiments, a drop is identified when solder and/or the wire form part of the circuitry for which resistance is being detected. Without wanting to be bound by theory, it is theorized that melting of the soldier and/or wire to the pad e.g., associated with improving electrical connection therebetween, reduces resistance from the joining tool through the pad.

Optionally, in some embodiments, upon the identifying, heating of the joining tool may be stopped. A potential advantage of accurate and/or early identification of establishment of a sufficiently good connection enables a reduced duration of heating and/or reduced heating temperature.

Alternatively, or additionally, in some embodiments, an initial resistance (pad and/or electrode) detected at an initial temperature and before activation of the joining tool is compared to a detected resistance after a joining process and after the flexible circuit strip has cooled to an initial temperature. Where the drop in resistance is identified in this comparison. In some embodiments, if this drop is insufficient, the joining process may be repeated e.g., to increase mechanical and/or electrical quality of the connection.

FIG. 4 is a simplified schematic plot of resistance with time, according to some embodiments of the disclosure.

In some embodiments, FIG. 4 illustrates detected resistance between a joining tool (e.g., joining tool 216) and a pad (e.g., pad 210a) to which the joining tool is establishing a connection. Where the resistance may be detected at the electrode as the "electrode resistance".

Where, initially at 400, prior the joining tool contacting the pad, detected resistance is high, for example, associated with electrically insulating material (e.g., air) between the joining tool and pad.

As the joining tool approaches the pad, detected resistance may drop 401 until contact is made at 402 between the pad and joining tool. Where one or both of a slope of the drop 401 and a lower resistance detected (e.g., below a threshold resistance and/or below a proportion of the initial resistance detected) are used to identify and/or verify contact between the joining tool and pad.

Heating of the joining tool during time period 404 may increase resistance detected, the increase, e.g., associated with increase in temperature of the flexible circuit strip. In some embodiments, for example, as described in method/s of FIG. 3A and/or FIG. 3B, heating of the joining tool may be controlled during time period 404 e.g., to maintain the resistance, R, below a threshold.

In some embodiments, when the connection is established resistance R may drop. Where a desired drop in resistance e.g., as illustrated at 406, in some embodiments, is associated with establishment of a good solder connection. In some embodiments, heating of the joining tool is ceased once the desired drop in resistance is identified. Where, in some embodiments, a peak resistance is identified, the value of which being used to determine the desired drop, which may be a given resistance or proportion of the peak resistance. In some embodiments, the desired drop is identified as resistance reaching (e.g., falling to) a given value after the identified peak.

In some embodiments, a final resistance detected 408 after heating (and optionally cooling) is used to assess quality of the connection. Where, in some embodiments, quality of the connection is verified when resistance detected is lower than a threshold and/or a proportion and/or value lower than that resistance detected prior 402 to performing the heat joining process.

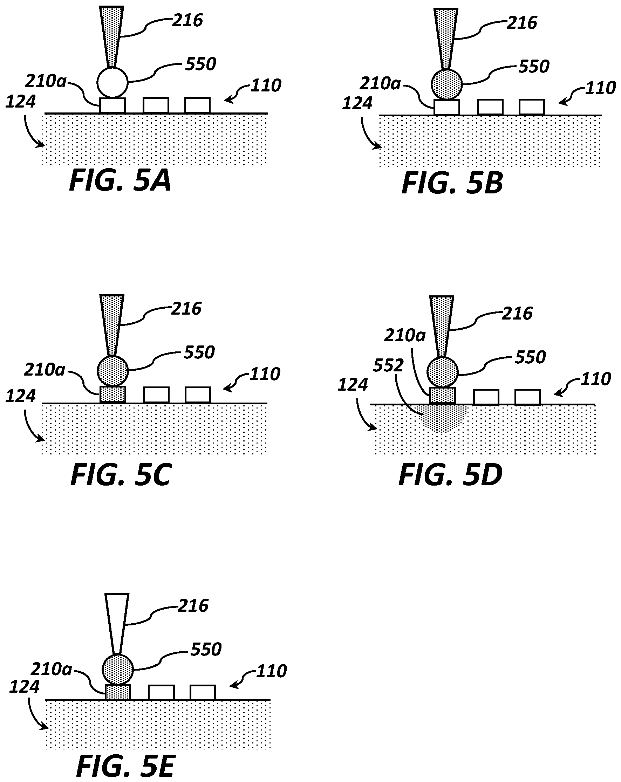

FIGs. 5A-E are simplified schematics illustrating establishing electrical connection to a pad 210a using a joining tool 216, according to some embodiments of the disclosure.

Where, in some embodiments, FIGs. 5A-E illustrate a portion of a flexible circuit strip 124 e.g., the same portion during establishing of electrical connection to pad 210a. In some embodiments, higher temperatures and/or heating is illustrated in FIGs. 5A-E by shading.

Referring now to FIG. 5A, in some embodiments, joining tool 216 (e.g., a tip of the joining tool) is brought into contact with a pad 210a and/or is brought into contact with wire 550 which is in contact with pad 210a. The joining tool is then heated, e.g., illustrated in FIG. 5A by shading of joining tool 216. In FIG. 5B, heat from joining tool 216 has conducted to wire 550 raising the temperature of wire 550. In some embodiments, heating of the wire 550 is sufficient to connect the wire to the pad via welding.

In some embodiments, heating of joining tool 216 continues, e.g., as illustrated in FIG. 5C where heat has been conducted to pad 210a raising the temperature of the pad. In some embodiments, this heating of both the wire and pad is sufficient to connect the wire to the pad via welding.

In some embodiments, the joining tool 216 is a soldering iron. Where one or both of the pad and wire may include solder and/or where solder may be positioned in contact with one or more of the pad, the joining tool, and the wire. The joining tool may contact one or more of the pad, wire, and solder material. Heating of the joining tool may then melt the solder to connect the pad and wire.

Referring now to FIG. 5D, heat has conducted from pad 210a to an upper portion 552 of flexible circuit strip 124 raising its temperature. With continued heating, raising of temperature of the upper layer of flexible circuit strip 124 e.g., adjacent to portion 552 may extend to pads neighboring pad 210a.

In some embodiments, this situation is avoided and/or temperature of the flexible circuit strip is maintained under a temperature at which damage occurs to flexible circuit strip 124 by sporadic (e.g., switched) heating of the joining tool. Switching potentially prevents over-heating of the flexible circuit strip material where the material is thermally insulating (e.g., in contrast with the pad material). Where, for example, prior to reaching the situation illustrated in FIG. 5D, joining tool heating is stopped e.g., power to heating element/s of the joining tool is cut e.g., as illustrated in FIG. 5E. Where, in some embodiments, the joining tool may be repetitively heated e.g., cycling to heat wire 550 and/or pad 210a to temperature/s associated with a good quality connection without damaging the flexible circuit strip.

### General

As used within this document, the term "about" refers to±20%

The terms "comprises", "comprising", "includes", "including", "having" and their conjugates mean "including but not limited to".

The term "consisting of" means "including and limited to".

As used herein, singular forms, for example, "a", "an" and "the" include plural references unless the context clearly dictates otherwise.

Within this application, various quantifications and/or expressions may include use of ranges. Range format should not be construed as an inflexible limitation on the scope of the present disclosure. Accordingly, descriptions including ranges should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within the stated range and/or subrange, for example, 1, 2, 3, 4, 5, and 6. Whenever a numerical range is indicated within this document, it is meant to include any cited numeral (fractional or integral) within the indicated range.

It is appreciated that certain features which are (e.g., for clarity) described in the context of separate embodiments, may also be provided in combination in a single embodiment. Where various features of the present disclosure, which are (e.g., for brevity) described in a context of a single embodiment, may also be provided separately or in any suitable sub-combination or may be suitable for use with any other described embodiment. Features described in the context of various embodiments are not to be considered essential features of those embodiments, unless the embodiment is inoperative without those elements.

Although the present disclosure has been described in conjunction with specific embodiments thereof, it is evident that many alternatives, modifications, and variations will be apparent to those skilled in the art. Accordingly, this application intends to embrace all such alternatives, modifications and variations that fall within the spirit and broad scope of the appended claims.

All references (e.g., publications, patents, patent applications) mentioned in this specification are herein incorporated in their entirety by reference into the specification, e.g., as if each individual publication, patent, or patent application was individually indicated to be incorporated herein by reference. Citation or identification of any reference in this application should not be construed as an admission that such reference is available as prior art to the present disclosure. In addition, any priority document(s) and/or documents related to this application (e.g., co-filed) are hereby incorporated herein by reference in its/their entirety.

Where section headings are used in this document, they should not be interpreted as necessarily limiting.

### GENERAL DESCRIPTION

Following is a non-exclusive list of some exemplary embodiments of the disclosure. The present disclosure also includes embodiments which include fewer than all the features in an example and embodiments using features from multiple examples, even if not listed below.

Example 1. A method of electrically connecting a wire (550) to a connection pad (210a) of a flexible circuit strip (124) using a heat joining tool (216), wherein the flexible circuit strip includes a polymer body (214), a plurality of electrodes (126), a plurality of connection pads (110) and a plurality of conductive traces (212), a trace connecting each of the plurality of electrodes (126) to a connection pad of the plurality of connection pads (110), the method comprising:
positioning said joining tool (216) in proximity to a connection pad (210a) of said plurality of connection pads (110) and said wire (550) to be connected to said connection pad (210a);
activating said joining tool (216) to heat a tip (238) of said joining tool (216) to electrically connect said wire (550) to said connection pad (210a);
monitoring, during said activating, a resistance between said joining tool (216) and an electrode (126a) electrically connected to said connection pad (210a), which resistance indicative of temperature of said flexible circuit strip (124); and
controlling said activating to maintain said resistance below an upper threshold associated with said flexible circuit strip (124) reaching a damaging temperature.

Example 2. The method according to Example 1, wherein said activating comprises supplying a plurality of electrical pulses to one or more heating element of said joining tool.

Example 3. The method according to Example 2, wherein said plurality of electrical pulses are voltage supply pulses to said one or more heating element.

Example 4. The method according to any one of Examples 2-3, wherein said controlling comprises controlling one or more features of said plurality of electrical pulses.

Example 5. The method according to Example 4, wherein said one or more features of said plurality of electrical pulses comprises one or more of:
a pulse duration, a pulse repetition frequency, and a pulse amplitude.

Example 6. The method according to Example 4, wherein said controlling one said one or more feature of said plurality of electrical pulses comprises pulse width modulation (PWM) of an activation signal to said joining tool (216).

Example 7. The method according to any one of Examples 1-4, wherein said controlling comprises maintaining said resistance above a lower threshold.

Example 8. The method according to any one of Examples 2-7, comprising determining a rate of change of said resistance;
wherein said controlling is using said rate of change.

Example 9. The method according to Example 8, wherein said determining comprises determining that said rate of change of said resistance is below a threshold; and
wherein said controlling comprises increasing an amplitude of said electrical power pulses.

Example 10. The method according to any one of Examples 2-9, wherein said controlling comprises:
identifying a portion of said resistance associated with connection between said wire (550) and said connection pad (210a); and
detecting a drop in said portion of said resistance.

Example 11. The method according to Example 10, comprising deactivating said joining tool (216) upon detecting said drop in said portion of said resistance.

Example 12. The method according to Example 11, wherein said detecting said drop in said resistance occurs during said supplying of a pulse of said plurality of electrical pulses.

Example 13. The method according to Example 10, comprising:
storing an initial measurement of said resistance prior to said activating when said flexible strip is at an initial temperature;
deactivating said joining tool (216); and
wherein said detecting said drop in said resistance comprises comparing said initial measurement of said resistance to a said resistance after said flexible strip has cooled to said initial temperature.

Example 14. The method according to any one of Examples 10-13, comprising assessing a quality of connection between said wire (550) and said connection pad (210a) based on said drop in said resistance.

Example 15. The method according to Example 14, wherein said quality is a quality of one or more of thermal, electrical, and mechanical connection between said wire and said connection pad.

Example 16. The method according to any one of Examples 14-15, comprising repeating said activating based on said assessing.

Example 17. The method according to any one of Examples 14-16, comprising adjusting one or more features of said supplying based on said assessing.

Example 18. A system for electrically connecting a wire (550) to a connection pad (210a) of a flexible circuit strip (124) including a polymer body (214), a plurality of electrodes (126), a plurality of connection pads (110) and a plurality of conductive traces (212), a trace connecting each of the plurality of electrodes (126) to a connection pad of the plurality of connection pads (110) comprising:
a heat joining tool (216) comprising one or more heating element;
a sensor (220) configured to measure a resistance between said joining tool (216) and an electrode (126a) electrically connected to a connection pad (210a) of said plurality of connection pads (110), which resistance indicative of temperature of said flexible circuit strip (124);
a controller (218) configured to:
   receive measurements of said resistance from said sensor (220); and
   control activation of said one or more heating element to heat a tip (238) of said joining tool (216) for connecting said connection pad (210a) and a wire (550), in proximity to said tip (238), while maintaining said resistance below an
   upper threshold associated with said flexible circuit strip (124) reaching a damaging temperature.

Example 19. A controller (218) for controlling electrically connecting of a wire (550) to a connection pad (210a) of a flexible circuit strip (124) using a heat joining tool (216), wherein the flexible circuit strip (124) includes a polymer body (214), a plurality of electrodes (126), a plurality of connection pads (110) and a plurality of conductive traces (212), a trace connecting each of the plurality of electrodes (126) to a connection pad of the plurality of connection pads (110), which controller (218) comprising circuitry configured to:
activate said joining tool to heat a tip (238) of said joining tool (216) to electrically connect said wire (550) to said connection pad (110a);
measure, during said activating, a resistance between said joining tool (216) and an electrode (126a) electrically connected to said connection pad (210a), which resistance indicative of temperature of said flexible circuit strip (124); and
control activation of said joining to maintain said resistance below an upper threshold associated with said flexible circuit strip (124) reaching a damaging temperature.

## Claims

1. A system for electrically connecting a wire (550) to a connection pad (210a) of a flexible circuit strip (124) including a polymer body (214), a plurality of electrodes (126), a plurality of connection pads (110) and a plurality of conductive traces (212), a trace connecting each of the plurality of electrodes (126) to a connection pad of the plurality of connection pads (110) comprising:
a heat joining tool (216) comprising one or more heating element;
a sensor (220) configured to measure a resistance between said joining tool (216) and an electrode (126a) electrically connected to a connection pad (210a) of said plurality of connection pads (110), which resistance indicative of temperature of said flexible circuit strip (124);
a controller (218) configured to:
receive measurements of said resistance from said sensor (220); and
control activation of said one or more heating element to heat a tip (238) of said joining tool (216) for connecting said connection pad (210a) and a wire (550), in proximity to said tip (238), while maintaining said resistance below an upper threshold associated with said flexible circuit strip (124) reaching a damaging temperature.

2. The system according to claim 1, wherein said controller (218) is configured to control activation by controlling supply of a plurality of electrical pulses to said one or more heating element.

3. The system according to claim 2, wherein said plurality of electrical pulses are voltage supply pulses to said one or more heating element.

4. The system according to any one of claims 2-3, wherein said controller (218) is configured to control one or more features of said plurality of electrical pulses.

5. The system according to claim 4, wherein said one or more features of said plurality of electrical pulses comprises one or more of: a pulse duration, a pulse repetition frequency, and a pulse amplitude.

6. The system according to claim 4, wherein said controller (218) is configured to control said one or more features of said plurality of electrical pulses by pulse width modulation (PWM) of an activation signal to said joining tool (216).

7. The system according to any one of claims 1-6, wherein said controller (218) is configured to maintain said resistance above a lower threshold.

8. The system according to any one of claims 2-7, wherein said controller (218) is configured to:
determine a rate of change of said resistance;
control said activation of said one or more heating element using said rate of change of said resistance.

9. The system according to claim 8, wherein said controller (218) is configured to
determine if said rate of change of said resistance is below a threshold; and
control said activation by increasing an amplitude of said plurality of electrical power pulses.

10. The system according to any one of claims 2-9, wherein controller (218) is configured to:
identify a portion of said resistance associated with connection between said wire (550) and said connection pad (210a);
detect a drop in said portion of said resistance; and
deactivate said joining tool (216) when said drop in said portion of said resistance is detected.

11. The system according to claim 10, wherein said controller (218) is configured to detect said drop in said resistance occurs during said supply of a pulse of said plurality of electrical pulses.

12. The system according to claim 10, wherein said controller (218) is configured to:
store an initial measurement of said resistance prior to activating said one or more heating element, when said flexible strip (124) is at an initial temperature;
deactivate said joining tool (216); and
wherein said controller is configured to detect said drop in said resistance by comparing said initial measurement of said resistance to a said resistance after said flexible strip (124) has cooled to said initial temperature.

13. The system according to any one of claims 10-12, wherein said controller is configured to assess a quality of connection between said wire (550) and said connection pad (210a) based on said drop in said resistance.

14. The system according to claim 13, wherein said controller (218) is configured to one or more of:
repeat activation of said one or more heating element based on said quality of connection; and
adjust one or more features of said supply of said plurality of electrical pulses, based on said quality of connection.

15. A method of electrically connecting a wire (550) to a connection pad (210a) of a flexible circuit strip (124) using a heat joining tool (216), wherein the flexible circuit strip includes a polymer body (214), a plurality of electrodes (126), a plurality of connection pads (110) and a plurality of conductive traces (212), a trace connecting each of the plurality of electrodes (126) to a connection pad of the plurality of connection pads (110), the method comprising:
positioning said joining tool (216) in proximity to a connection pad (210a) of said plurality of connection pads (110) and said wire (550) to be connected to said connection pad (210a);
activating said joining tool (216) to heat a tip (238) of said joining tool (216) to electrically connect said wire (550) to said connection pad (210a);
monitoring, during said activating, a resistance between said joining tool (216) and an electrode (126a) electrically connected to said connection pad (210a), which resistance indicative of temperature of said flexible circuit strip (124); and
controlling said activating to maintain said resistance below an upper threshold associated with said flexible circuit strip (124) reaching a damaging temperature.
